# EUROPÄISCHE PATENTANMELDUNG

(11) **EP 4 659 691 A1**
(43) Veröffentlichungstag der Anmeldung: **10.12.2025**
(21) Anmeldenummer: 25180339.1
(22) Anmeldetag: 03.06.2025
(51) Int. Cl.: A61B 17/34, A61B 90/00

(54) **MEDIZINISCHES AR-SYSTEM FÜR EINEN CHIRURGISCHEN EINGRIFF UND VERFAHREN ZUR ÜBERPRÜFUNG EINER NAVIGATIONSGENAUIGKEIT**

(30) Priorität: 04.06.2024 DE 102024115529
(71) Anmelder: B. Braun New Ventures GmbH, 79110 Freiburg im Breisgau (DE)
(72) Erfinder: DIPPEL, Maximilian, 79115 Freiburg (DE); STAWIASKI, Jean, 79117 Freiburg (DE)
(74) Vertreter: Winter, Brandl - Partnerschaft mbB

(57) **Zusammenfassung**

Die Offenbarung betrifft ein Medizinisches AR-System (1) für einen chirurgischen Eingriff eines Patienten (10), mit einer Visualisierungseinheit (2), zum Erzeugen einer präoperativen Aufnahme und einer zeitaktuellen Aufnahme mit einem Referenzpunkt (18), einem Navigationssystem (8) zum Erfassen der Visualisierungseinheit (2), und einer Steuereinheit (16) zum Erzeugen einer AR-Überlagerungsdarstellung aus einem digitalisierten Referenzpunkt (20) aus der präoperativen Aufnahme und der zeitaktuellen Aufnahme und zum Darstellen eines Vergleichs der AR-Überlagerungsdarstellung. Daneben betrifft die Offenbarung ein Verfahren zur Überprüfung einer Navigationsgenauigkeit des medizinischen AR-Systems (1) sowie ein computerlesbares Speichermedium und/oder ein Computerprogramm.

## Beschreibung

### Technisches Gebiet

Die vorliegende Offenbarung betrifft ein medizinisches Augmented-Reality(AR)-System für einen chirurgischen, insbesondere neurochirurgischen, Eingriff eines Patienten. Das AR-System weist eine Visualisierungseinheit, die eine präoperative Aufnahme, insbesondere eine 3D-Aufnahme, des Patienten mit zumindest einem auf diesem markierten (aktuellen) Referenzpunkt und eine zeitaktuelle Aufnahme, insbesondere eine 3D-Aufnahme, des Patienten mit dem zumindest einen (aktuellen) Referenzpunkt erzeugt. Zudem weist das AR-System eine Steuereinheit auf, die eingerichtet ist, einen auf den Patienten registrierten digitalisierten Referenzpunkt aus dem zumindest einen (aktuellen) Referenzpunkt der präoperativen Aufnahme zu generieren. Daneben betrifft die vorliegende Offenbarung ein Verfahren zur Überprüfung einer Navigationsgenauigkeit des medizinischen AR-Systems sowie ein computerlesbares Speichermedium und ein Computerprogramm.

### Hintergrund der Offenbarung

Traditionelle Ansätze in der chirurgischen Navigation und Planung stützen sich auf präoperative Bildgebungsverfahren, um detaillierte Darstellungen der chirurgischen Zielgebiete zu erhalten. Diese Methoden umfassen typischerweise die Verwendung von MRI (Magnetresonanztomographie), CT (Computertomographie) und Ultraschallbildern, um umfassende Ansichten der zu operierenden Bereiche zu generieren. Trotz der hohen Qualität dieser Bilder besteht eine Herausforderung darin, diese präoperativen Bilder effektiv mit der tatsächlichen Situation während des chirurgischen Eingriffs zu verknüpfen. Bekannte Systeme setzen auf externe Tracking-Systeme, um die präoperativ erzeugten Aufnahmen und der intraoperativen Realität zu verbinden. Diese Ansätze erfordern jedoch oft umständliche Setup-Prozesse und können die Bewegungsfreiheit des Chirurgen einschränken.

Auf dem Gebiet der medizinischen Bildgebung und Chirurgie hat die Integration von Augmented Reality (AR)-Technologien bedeutende Fortschritte ermöglicht, insbesondere in der Präzision und Effizienz chirurgischer Eingriffe. Augmented Reality bietet eine berührungslose Möglichkeit, mit Patientendaten zu interagieren und diese wahrzunehmen. Mit der Einführung von AR in den Operationssaal haben sich neue Möglichkeiten eröffnet, die eine direktere und intuitivere Interaktion mit präoperativen Daten ermöglichen. Durch die Überlagerung digitaler Bilder direkt auf das Sichtfeld des Chirurgen können AR-Systeme eine nahtlose Integration von Bildgebungsinformationen in den chirurgischen Workflow bieten. Trotz dieser beträchtlichen Fortschritte gibt es nach wie vor Einschränkungen in Bezug auf die Genauigkeit der Überlagerung und die Fähigkeit, Veränderungen in Echtzeit zu berücksichtigen. Die Genauigkeit der Positionsbestimmung und die Stabilität der Bildüberlagerung sind kritische Faktoren, die die Nützlichkeit von AR in der Chirurgie beeinflussen. Insbesondere in hochpräzisen Anwendungen wie der Neurochirurgie können geringfügige Ungenauigkeiten in dem chirurgischen Verfahren erhebliche Auswirkungen mit teils katastrophalen Folgen hinsichtlich der Patientensicherheit haben. Zusätzlich erfordert die effektive Nutzung von AR-Systemen in der Chirurgie eine nahtlose Integration in den chirurgischen Workflow, was eine Herausforderung darstellt, da die Systeme oft eine zusätzliche kognitive Belastung für den Chirurgen darstellen und die Interaktion mit den Systemen während des Eingriffs intuitiv und störungsfrei sein muss.

Herkömmliche chirurgische (AR-)Assistenzsysteme sind beispielsweise in der US 2013 / 0 293 578 A1, US 2022 / 0 215 532 A1 oder CN 1 12 043 382 A beschrieben.

Trotz der beträchtlichen Fortschritte auf dem Gebiet der medizinischen Bildgebung und AR-Technologie besteht nach wie vor ein Bedarf an verbesserten Methoden und Systemen, die eine zuverlässige und benutzerfreundliche Integration von AR in chirurgische Verfahren ermöglichen, ohne nachteilige Effekte auf die Patientensicherheit zu generieren. Insbesondere besteht ein Bedarf an Systemen, die eine höhere Navigationsgenauigkeit und eine bessere Anpassungsfähigkeit an intraoperative Veränderungen bieten, ohne den chirurgischen Workflow zu unterbrechen oder die kognitive Belastung des Chirurgen zu erhöhen.

### Zusammenfassung der Offenbarung

Daher liegt der vorliegenden Offenbarung die Aufgabe zugrunde, die vorstehend beschriebenen Nachteile zu vermeiden oder wenigstens abzumildern und insbesondere ein medizinisches AR-System sowie ein Verfahren bereitzustellen, welches eine präzise und zuverlässige Integration von AR in chirurgische Verfahren ermöglicht.

Diese Aufgabe wird durch ein medizinisches AR-System gemäß den Merkmalen des Anspruchs 1, durch ein Verfahren zum Überprüfen einer Navigationsgenauigkeit bzw. durch ein computerlesbares Speichermedium und/oder ein Computerprogramm gemäß den Merkmalen der nebengeordneten Patentansprüche gelöst. Vorteilhafte Ausführungsformen sind in den Unteransprüchen beansprucht und/oder werden nachfolgend erläutert.

Die Offenbarung betrifft demzufolge zunächst ein medizinisches AR-System für einen chirurgischen, insbesondere neurochirurgischen, Eingriff eines Patienten. Das medizinische AR-System weist eine Visualisierungseinheit auf.

Die Visualisierungseinheit ist ausgebildet, um eine präoperative (3D-)Aufnahme des Patienten mit zumindest einem auf diesem (d.h. auf dem Patienten) markierten (aktuellen/momentanen) Referenzpunkt zu erzeugen und bereitzustellen. Die Visualisierungseinheit ist ausgebildet, um eine zeitaktuelle (3D-)Aufnahme des Patienten, beispielsweise in Form eines Video-Feeds, mit dem zumindest einem (aktuellen) Referenzpunkt/Markierung zu erzeugen und bereitzustellen. Das medizinische AR-System weist ein Navigationssystem auf. Das Navigationssystem ist dafür ausgebildet, eine Position und Orientierung der Visualisierungseinheit in einem globalen Koordinatensystem gegenüber dem Patienten zu erfassen. Das medizinische AR-System weist eine visuelle Darstellungsvorrichtung auf, um visuelle Informationen für den chirurgischen Eingriff darzustellen. Das medizinische AR-System eine Steuereinheit auf. Die Steuereinheit ist eingerichtet und vorbereitet, zumindest einen auf den Patienten registrierten digitalisierten Referenzpunkt aus dem zumindest einen (aktuellen) Referenzpunkt der präoperativen (3D-)Aufnahme des Patienten zu generieren und abzuspeichern. Dabei ist die Steuereinheit zusätzlich eingerichtet und vorbereitet ist, eine AR-Überlagerungsdarstellung mit dem zumindest einen digitalisierten Referenzpunkt und der zeitaktuellen Aufnahme des Patienten, (welche den zumindest einen aktuellen Referenzpunkt aufweist) zu erzeugen, diese auf der visuellen Darstellungsvorrichtung darzustellen, und den zumindest einen digitalisierten Referenzpunkt mit dem zumindest einen (aktuellen) Referenzpunkt der zeitaktuellen Aufnahme zu vergleichen. Dies dient insbesondere dazu, um eine Navigationsgenauigkeit des medizinischen AR-Systems während des Eingriffs zu überprüfen.

In anderen Worten ausgedrückt, weist das System eine Visualisierungseinheit, die in Echtzeit zeitaktuelle Aufnahmen des Patienten mit zumindest einem, vorzugsweise mindestens zwei oder drei, markierten aktuellen Referenzpunkten und eine präoperative momentane 3D-Aufnahme des Patienten und dem zumindest einen, vorzugsweise mindestens zwei oder drei, aktuellen Referenzpunkt erzeugt. Die Visualisierungseinheit des medizinischen AR-Systems ist ausgebildet, die präoperative 3D-Aufnahme des Patienten inklusive dem mindestens einen aktuellen Referenzpunkt in dieser präoperativen 3D-Aufnahme zu generieren, welche anschließend einem Navigationssystem übermittelt werden und welche als Basis der Navigationsprozesse im nachfolgenden chirurgischen Eingriff dienen. Ein Navigationssystem erfasst die Position und Orientierung der Visualisierungseinheit im Verhältnis zum Patienten in einem globalen Koordinatensystem. Die visuelle Darstellungsvorrichtung zeigt visuelle Informationen für den chirurgischen Eingriff an, während eine Steuereinheit digitale Referenzpunkte aus den aktuellen Referenzpunkten der präoperativen Aufnahme generiert und speichert.

Gemäß einem zentralen Aspekt der Offenbarung erzeugt die Steuereinheit eine AR-Überlagerungsdarstellung, die die digitalisierten Referenzpunkte mit den aktuellen Referenzpunkten der Echtzeitaufnahme des Patienten gleichzeitig (überlagert) darstellt. Dies ermöglicht es dem Nutzer oder vorzugsweise dem System, die Navigationsgenauigkeit während des Eingriffs kontinuierlich zu überprüfen und (automatisch) anzupassen. Eine visuelle Darstellungsvorrichtung dient der Anzeige dieser visuellen Informationen und stellt die augmentierten Bilder und Daten in einer für den Nutzer verständlichen und nutzbaren Form dar. Die Fähigkeit, Echtzeitdaten und -bilder nahtlos in das Sichtfeld des Chirurgen zu integrieren, verbessert die Entscheidungsfindung und Präzision während des Eingriffs. Die Steuereinheit ist für die Generierung und Speicherung von digitalisierten Referenzpunkten aus den präoperativen 3D-Aufnahmen des Patienten eingerichtet. Diese digitalisierten Punkte dienen als Anker für die Überlagerung der AR-Darstellungen und sind entscheidend für die Aufrechterhaltung der räumlichen Konsistenz zwischen den virtuellen Informationen und dem physischen Patienten. Die Fähigkeit, diese Referenzpunkte präzise zu generieren und zu speichern, ist grundlegend für die Genauigkeit und Zuverlässigkeit des gesamten Systems.

Durch eine, insbesondere kontaktlose, Echtzeitüberprüfung der Navigationsgenauigkeit wird die chirurgischen Präzision verbessert, was wiederum die Sicherheit und Effektivität des Eingriffs erhöht und die Herausforderung der Aufrechterhaltung der Genauigkeit in dynamischen, operativen Umgebungen angeht. Diese hochauflösenden 3D-Bilder sind grundlegend für die Planung und Ausführung des Eingriffs, indem sie eine dreidimensionale Basis für die Navigation und Orientierung bieten. Die Referenzpunkte präoperativen Aufnahmen werden mit den aktuellen Bildern, die während des Eingriffs aufgenommen werden, abgeglichen, um eine kontinuierliche Genauigkeit und Aktualität der visuellen Informationen zu gewährleisten. Somit können Planungs- und Navigationsinformationen in allen Phasen eines chirurgischen Eingriffs vermittelt werden, so dass der Anwender mithilfe von Augmented Reality und mit gleichzeitiger geringer manueller Interaktion die Genauigkeit der Registrierung und Navigation von medizinischen Systemen, insbesondere während des Eingriffsprozesses, überprüfen kann.

In einer weiteren bevorzugten Ausführungsform der Offenbarung kann die Steuereinheit eingerichtet und vorbereitet sein, einen Abstand zwischen dem zumindest einen digitalisierten Referenzpunkt und dem zumindest einen aktuellen Referenzpunkt der zeitaktuellen Aufnahme als einen Navigationsfehler des medizinischen AR-Systems zu ermitteln und vorzugsweise automatisch zu korrigieren.

Anders ausgedrückt, kann die Steuereinheit, insbesondere bevor der eigentliche chirurgische Eingriff startet, eine Abweichung zwischen den digitalisierten Referenzpunkten und den aktuellen Referenzpunkten am Patienten der aktuellen (Video-Feed-)Aufnahme ermitteln und vorzugsweise quantifizieren und diese auf der Darstellungsvorrichtung auszugeben/anzuzeigen.

Dies ermöglicht eine präzisere und dynamischere Anpassung und Überprüfung der Navigationsgenauigkeit während des chirurgischen Eingriffs. Die Steuereinheit, die bereits für die Erzeugung einer AR-Überlagerungsdarstellung mit dem digitalisierten Referenzpunkt und der zeitaktuellen Aufnahme des Patienten, welche den aktuellen Referenzpunkt aufweist, konfiguriert ist, ist somit in der Lage, die räumliche Diskrepanz zwischen dem digitalisierten und dem aktuellen Referenzpunkt zu quantifizieren. Diese Quantifizierung des Abstands ermöglicht es dem System, eine objektive Messung der Abweichung in Echtzeit zu liefern, was eine Korrektur/Ausgleichen durch den Nutzer ermöglicht, insbesondere bevor der chirurgische Eingriff beginnt. Die Identifikation und Quantifizierung des Navigationsfehlers als spezifische Abstandsmessung zwischen den Referenzpunkten stellt eine direkte Feedback-Schleife zur Verfügung, die es dem chirurgischen Team ermöglicht, die Präzision des Eingriffs zu überwachen und anzupassen. Die Implementierung dieser spezifischen Mechanismen der Kommunikation zwischen den Komponenten, insbesondere zwischen der Steuereinheit und der visuellen Darstellungsvorrichtung, ermöglicht eine nahtlose Integration der Echtzeit-Überwachung der Navigationsgenauigkeit in den Workflow des chirurgischen Eingriffs. Diese Innovation führt zu einer erhöhten Sicherheit und Effizienz bei chirurgischen Eingriffen, indem sie die Risiken von Navigationsfehlern minimiert und eine präzisere Ausrichtung auf die chirurgischen Ziele ermöglicht. Die Fähigkeit, Navigationsfehler, vorzugsweise in Echtzeit, zu identifizieren und zu korrigieren, repräsentiert einen bedeutenden Fortschritt in der medizinischen Bildgebung und Navigation, der das Potenzial hat, die Ergebnisse für Patienten signifikant zu verbessern und die Herausforderungen, denen Chirurgen bei komplexen Eingriffen gegenüberstehen, zu verringern.

In einem weiteren vorteilhaften Aspekt der Offenbarung kann das Navigationssystem ausgebildet sein, (zusätzlich) eine Position und Orientierung einer medizinischen Instrumentenspitze in einem globalen Koordinatensystem gegenüber dem Patienten zu erfassen und die Steuereinheit eingerichtet und ausgebildet ist, den zumindest einen digitalisierten Referenzpunkt mithilfe der Position und Orientierung der medizinischen Instrumentenspitze zu generieren.

Diese Erweiterung ermöglicht eine direkte und präzise Verfolgung der Instrumentenspitze in Echtzeit, welche beispielsweise mit einem eigenen Marker/Tracker ausgebildet ist. Die Steuereinheit kann eingerichtet sein, einen digitalisierten Referenzpunkt mithilfe der Position und Orientierung der medizinischen Instrumentenspitze zu generieren. Dies ermöglicht es, die Position der Instrumentenspitze in Bezug auf die präoperativen Planungsdaten und die aktuellen intraoperativen Bedingungen zu aktualisieren und zu verfeinern und zu digitalisierende Referenzpunkte am Patienten mit der Instrumentenspitze auszuwählen.

Alternativ oder zusätzlich kann die Steuereinheit eingerichtet und vorbereitet sein, die Referenzpunkte mithilfe eines Laserpointers und/oder einem Fokuspunkt der Visualisierungseinheit auszuwählen und zu digitalisieren. Der Einsatz eines Laserpointers oder eines Fokuspunkts ermöglicht eine deutlich präzisere Lokalisierung und Auswahl der Referenzpunkte auf dem Patienten. Der Fokuspunkt, der Teil der Visualisierungseinheit ist, erlaubt es, die Positionierung der Referenzpunkte mit hoher Genauigkeit durchzuführen, indem er direkt auf die relevante Stelle des Patientenkörpers gerichtet wird. Demnach kann die Steuereinheit einen digitalisierten Referenzpunkt erzeugen, der dann in die Augmented-Reality(AR)-Überlagerungsdarstellung eingespeist wird. Diese Darstellung, die auf der visuellen Darstellungsvorrichtung angezeigt wird, kombiniert die digitale Information mit der realen Welt.

In einer weiteren oder alternativen vorteilhaften Ausführungsform der Offenbarung kann die Steuereinheit eingerichtet und ausgebildet ist sein, den zumindest einen digitalisierten Referenzpunkt mithilfe des zumindest einen aktuellen Referenzpunkts der von der Visualisierungseinheit erzeugten präoperativen 3D-Aufnahme des Patienten automatisch zu generieren. Das heißt, die Steuereinheit kann eingerichtet sein, digitalisierte Referenzpunkte anhand der von der Visualisierungseinheit erzeugten präoperativen 3D-Aufnahme des Patienten und der in dieser Aufnahme am Patienten angeordneten aktuellen Referenzpunkte/Markierungen automatisch, das heißt ohne eine zusätzliche manuelle Auswahl der aktuellen Referenzpunkte am Patienten, zu erzeugen.

Vorteilhafterweise ist somit eine automatische Generierung des zumindest einen digitalisierten Referenzpunkts durch die Steuereinheit möglich, basierend auf dem aktuellen Referenzpunkt, der in der präoperativen 3D-Aufnahme identifiziert wurde.

In einer weiteren bevorzugten Ausführungsform der Offenbarung kann das medizinische AR-System ein AR-Headset und/oder ein 2D-Monitor und/oder ein 3D-Monitor und/oder ein VR-Headset als die Darstellungsvorrichtung aufweisen.

In anderen Worten, ist die visuelle AR-Überlagerungsdarstellung aus der zeitaktuellen Aufnahme und der digitalisierten Referenzpunkte alternativ oder ergänzend über ein VR- oder AR-Headset, welches der Nutzer auf dem Kopf trägt, anzeigbar.

Eine Integration eines AR-Headsets bzw. eines VR-Headsets als Darstellungsvorrichtung ermöglicht es dem Nutzer, eine immersive, augmentierte Realitätserfahrung zu erhalten, bei der digitale Informationen direkt in das Sichtfeld des Benutzers eingeblendet werden. Dies fördert eine intuitive Interaktion mit den visuellen Daten und unterstützt eine präzisere Navigation und Orientierung während des chirurgischen Eingriffs, indem es die Notwendigkeit verringert, den Blick von der Operationsstelle zu lösen. Die Verwendung eines 3D-Monitors als Darstellungsvorrichtung bietet eine alternative Visualisierungsmethode, die es dem Operationsteam ermöglicht, die dreidimensionalen anatomischen Strukturen und die Positionierung der Referenzpunkte in Echtzeit zu betrachten, was eine kollektive Beurteilung und Entscheidungsfindung während des Eingriffs erleichtert.

In einer weiteren bevorzugten Ausführungsform der Offenbarung kann das Navigationssystem ein infrarotbasiertes Trackingsystem oder ein elektromagnetisches Trackingsystem oder ein optisches Machine-Vision Trackingsystem aufweisen.

Anders ausgedrückt, erfolgt das Erfassen der Visualisierungseinheit insbesondere über das Navigationssystem bspw. mithilfe eines infrarotbasierten oder elektromagnetischen oder optischen Trackingsystems. In allen Fällen erfasst das Trackingsystem die relative Lage des Visualisierungssystems zum Patienten. Ebenfalls ist ein Trackingsystem einsetzbar, welches die relative Position des Visualisierungssystems zum Patienten aus kinematischen Daten/ Informationen des Roboters bzw. des Roboterarms, mit welchem die Visualisierungseinheit in Verbindung steht, berechnet.

Nochmals anders ausgedrückt, kann das Navigationssystem entweder ein infrarotbasiertes Trackingsystem, ein elektromagnetisches Trackingsystem oder ein optisches Machine-Vision Trackingsystem sein. Bei einem infrarotbasierten Trackingsystem erfolgt die Kommunikation durch Infrarotsignale, die von Markern reflektiert werden, die am Patienten angebracht sind, wodurch eine hochpräzise Positionierung und Orientierung der Visualisierungseinheit relativ zum Patienten ermöglicht wird. Ein elektromagnetisches Trackingsystem nutzt elektromagnetische Felder, um die räumliche Position und Orientierung der Visualisierungseinheit zu erfassen. Ein optisches Machine-Vision Trackingsystem hingegen verwendet Bildverarbeitungsalgorithmen, um die Position der Markierungen auf dem Patienten zu erkennen und zu verfolgen.

Gemäß einem weiteren vorteilhaften Aspekt der Offenbarung kann die Visualisierungseinheit als ein Operationsmikroskop, ein chirurgisches Exoskop, ein chirurgisches Endoskop oder als eine optische Kamera ausgebildet sein.

Vorzugweise ermöglicht kann das erfindungsgemäße AR-System eine 3D-Wahrnehmung der angezeigten AR-Informationen ermöglichen. Weiter vorzugsweise kann das gesamte AR-System in einer simulierten Umgebung oder in einem digitalen Zwilling laufen.

Die vorliegende Offenbarung betrifft weiterhin ein Verfahren zur Überprüfung einer Navigationsgenauigkeit eines medizinischen AR-Systems, mit den Schritten Setzen zumindest eines, vorzugsweise mindestens zweier, aktuellen Referenzpunkts/Markierung auf einen Patienten, Erzeugen und Bereitstellen einer präoperativen 3D-Aufnahme des Patienten mit dem zumindest einen aktuellen Referenzpunkt durch eine Visualisierungseinheit, Generieren und Abspeichern zumindest eines digitalisierten Referenzpunkts aus dem zumindest einen aktuellen Referenzpunkt der präoperativen 3D-Aufnahme des Patienten, durch eine Steuereinheit, Erzeugen und Bereitstellen einer zeitaktuellen Aufnahme des Patienten mit dem zumindest einen aktuellen Referenzpunkt durch die Visualisierungseinheit, Erzeugen einer AR-Überlagerungsdarstellung mit dem zumindest einen digitalisierten Referenzpunkt und der zeitaktuellen Aufnahme des Patienten, welche den zumindest einen aktuellen Referenzpunkt aufweist, Ausgeben der AR-Überlagerungsdarstellung durch eine visuelle Darstellungsvorrichtung, und Vergleichen des zumindest einen digitalisierten Referenzpunkts mit dem zumindest einen aktuellen Referenzpunkt der zeitaktuellen Aufnahme, um eine Navigationsgenauigkeit des medizinischen AR-Systems zu überprüfen.

Gemäß einer weiteren vorteilhaften Ausführungsform der Offenbarung kann das Verfahren einen zusätzlichen Schritt Berechnen eines Navigationsfehlers des medizinischen AR-Systems aus einem Abstand/Abweichung zwischen dem zumindest einen digitalisierten Referenzpunkt und dem zumindest einen aktuellen Referenzpunkt der zeitaktuellen Aufnahme aufweisen.

Durch die Berechnung des Abstands zwischen den jeweiligen Referenzpunkten kann die Steuereinheit einen Navigationsfehler ermitteln. Die Fähigkeit, Abweichungen sofort zu erkennen und zu korrigieren, minimiert das Risiko von Fehlern während des Eingriffs, verbessert das chirurgische Ergebnis und erhöht die Patientensicherheit. Zudem ermöglicht dieses Merkmal eine objektive Bewertung der Leistungsfähigkeit des medizinischen AR-Systems, indem es eine quantifizierbare Messung der Navigationsgenauigkeit liefert.

Gemäß einer weiteren Ausführungsform kann das Navigationssystem eine Position und Orientierung einer medizinischen Instrumentenspitze in einem globalen Koordinatensystem gegenüber dem Patienten erfassen und der Schritt Generieren und Abspeichern des zumindest einen digitalisierten Referenzpunkts kann mithilfe der Position und Orientierung der medizinischen Instrumentenspitze durchgeführt werden.

Die vorliegende Offenbarung betrifft weiterhin ein computerlesbares Speichermedium, umfassend Befehle, die bei einer Ausführung durch einen Computer diesen veranlassen, die offenbarungsgemäßen Verfahrensschritte auszuführen.

Ferner betrifft die vorliegende Offenbarung weiterhin ein Computerprogramm mit Anweisungen, bei deren Ausführung durch einen Computer, den Computer veranlasst, die offenbarungsgemäßen Verfahrensschritte durchzuführen.

### Kurzbeschreibung der Figuren

Die Offenbarung wird nachfolgend anhand bevorzugter Ausführungsformen mit Hilfe der begleitenden Figuren näher erläutert. Es zeigen:
Fig. 1 eine beispielhafte perspektivische Seitenansicht eines medizinischen AR-Systems gemäß einem bevorzugten Ausführungsbeispiel der vorliegenden Offenbarung;
Fig. 2 eine beispielhafte zeitaktuelle Aufnahme eines Patienten mit einer Mehrzahl von auf diesem markierten Referenzpunkten und eines beispielhaften medizinischen Instruments gemäß dem bevorzugten Ausführungsbeispiel der vorliegenden Offenbarung;
Fig. 3 einen beispielhaften Erfassungsvorgang einer präoperativen 3D-Aufnahme des Patienten und der Mehrzahl von markierten Referenzpunkten durch eine Visualisierungseinheit, welche dem Navigationssystem automatisch als digitalisierte Referenzpunkte übermittelt werden, gemäß dem bevorzugten Ausführungsbeispiel der vorliegenden Offenbarung;
Fig. 4 eine beispielhafte perspektivische AR-Überlagerungsdarstellung mit einer Mehrzahl von digitalisierten Referenzpunkten und der zeitaktuellen Aufnahme eines drapierten Patienten, und
Fig. 5 ein Flussdiagramm eines Verfahrens zur Überprüfung einer Navigationsgenauigkeit des medizinischen AR-Systems gemäß einer bevorzugten Ausführungsform der vorliegenden Offenbarung.

Die Figuren sind schematischer Natur und dienen lediglich dem Verständnis der Offenbarung. Gleiche Elemente sind mit denselben Bezugszeichen versehen. Die Merkmale der verschiedenen Ausführungsformen können untereinander ausgetauscht werden sowie in einer beliebigen Kombination auftreten.

### Beschreibung der Ausführungsbeispiele

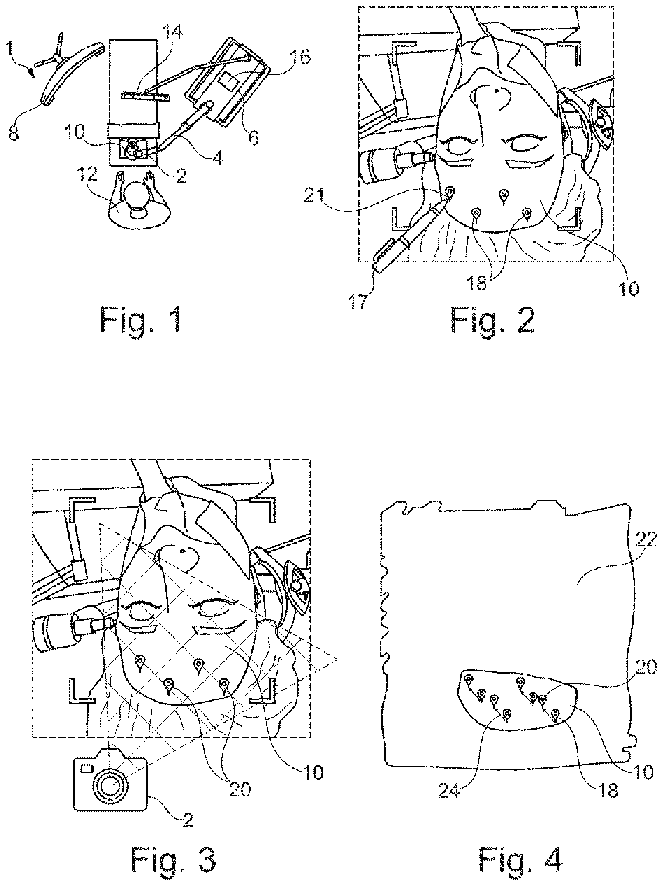

Nachfolgend wird die vorliegende Offenbarung anhand einer vorteilhaften Ausführungsform mit Bezug auf die Figuren 1 bis 5 beschrieben.

Figur 1 ist eine beispielhafte perspektivische Seitenansicht eines medizinischen AR-Systems 1 gemäß einem bevorzugten Ausführungsbeispiel der vorliegenden Offenbarung. Das AR-System 1 weist eine bewegliche Visualisierungseinheit 2 auf, insbesondere in der Form eines Operationsmikroskops auf, welche mit einem beweglichen Roboterarm 4 eines medizinischen Roboters 6 in Verbindung steht, um mittels Steuerung des Roboterarms 4 sowohl eine Position (x,y,z) als auch eine Orientierung der Visualisierungseinheit 2 im Raum gegenüber dem Patienten einzustellen. Der Nutzer/ Operator 12 kontrolliert die Lage der Visualisierungseinheit 2, welche eine zeitaktuelle Aufnahme von einem Patienten 10 sowie von präoperativ gesetzten Referenzpunkten/Markierungen auf dem Patienten erzeugt. Ferner, weist das AR-System 1 ein Navigationssystem 8 auf, welches mittels einer integrierten Navigationskamera, vorliegend einer Stereokamera, die Position sowie die Orientierung der Visualisierungseinheit 2 relativ zu einem (registrierten) Patienten 10 erfasst. Das AR-System 1 weist außerdem eine Darstellungsvorrichtung 14, beispielsweise in Form eines 2D-Monitors, sowie eine Steuereinheit 16 auf. Die Steuereinheit 16 ist derart angepasst, dass sie eine AR-Überlagerungsdarstellung mit zumindest einem digitalisierten Referenzpunkt 20 und der zeitaktuellen Aufnahme des Patienten 10, welche den zumindest einen aktuellen Referenzpunkt 18 aufweist, generiert und auf der Darstellungsvorrichtung 14 darstellt.

Figur 2 ist eine beispielhafte zeitaktuelle Aufnahme des Patienten 10 mit einer Mehrzahl von auf diesem markierten (aktuellen) Referenzpunkten 18 und eines beispielhaften medizinischen Instruments 17, welche eine vom Navigationssystem 8 trackbare/verfolgbare Instrumentenspitze 21 aufweist, gemäß dem bevorzugten Ausführungsbeispiel der vorliegenden Offenbarung. Die zeitaktuelle Aufnahme des Patienten 10, welche durch die Visualisierungseinheit 2 erfasst wird und als zeitaktuelle Videoübertragung auf der Darstellungsvorrichtung 14 anzeigbar ist, stellt somit ein zeitaktuelles Bild des Patienten 10 inklusive der zeitaktuellen/realitätstreuen Markierungen/Referenzpunkte 18 und vorzugsweise auch ein zeitaktuelles Bild eines medizinischen Instruments 17 dar.

Figur 3 zeigt einen beispielhaften Erfassungsvorgang einer präoperativen 3D-Aufnahme des Patienten 10 und der Mehrzahl von markierten Referenzpunkten 18 durch eine Visualisierungseinheit 2, welche einem Navigationssystem 8 automatisch als digitalisierte Referenzpunkte 20 übermittelt werden, gemäß dem bevorzugten Ausführungsbeispiel der vorliegenden Offenbarung. Dabei werden die digitalisierten Referenzpunkte 20 anhand der erfassten zeitaktuellen Referenzpunkte 18 durch die Steuereinheit 16 des medizinischen AR-Systems 1 generiert.

Figur 4 ist eine beispielhafte perspektivische AR-Überlagerungsdarstellungen mit einer Mehrzahl von digitalisierten Referenzpunkten 20 und der zeitaktuellen Aufnahme eines Patienten 10 mit einer Mehrzahl von zeitaktuellen Referenzpunkten 18. In dieser Darstellung ist der Patient 10 für einen nachfolgenden chirurgischen Eingriff durch eine medizinische Plane/Abdeckung 22 drapiert und nur ein Teil (in dieser Darstellung die Stirn) des Patienten 10, welcher die Referenzpunkte 18 aufweist, ist nicht von der medizinischen Plane 22 abgedeckt. Die charakteristischen anatomischen Orientierungspunkte des Patienten 10 sind somit nicht mehr leicht für die Visualisierungseinheit 2 zugänglich. Dennoch ist es möglich, mithilfe von Augmented Reality die zuvor digitalisierten Referenzpunkte 20 dem Nutzer 12 anzuzeigen, der dann in der Lage ist, diese visuell mit den auf dem Patienten 10 markierten und zeitaktuell in der Darstellungsvorrichtung 14 angezeigten Referenzpunkte 18 zu vergleichen. Die Verschiebung zwischen den digitalisierten Referenzpunkten 20 und den jeweiligen zeitaktuellen Referenzpunkten 18 ist dann der Navigationsfehler 24 des AR-Systems 1, welcher vorzugsweise separat auf der Darstellungsvorrichtung 14 angedeutet bzw. hervorgehoben wird.

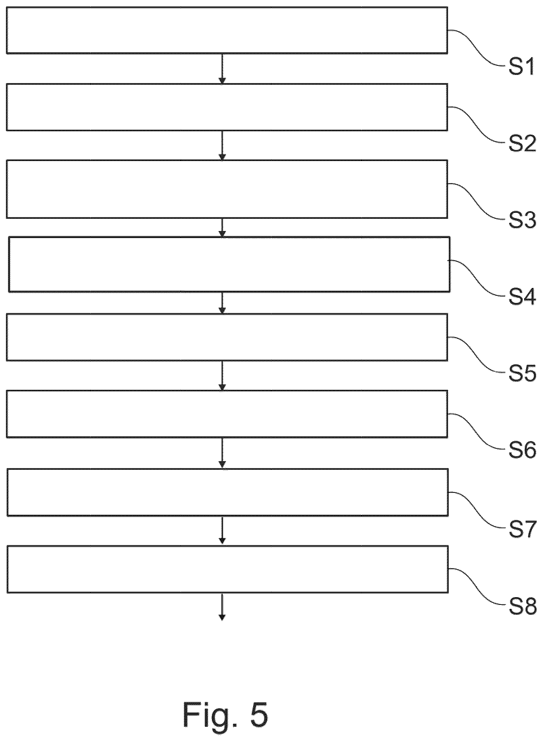

Figur 5 ist ein Flussdiagramm eines (computerimplementierten) Verfahrens zur Überprüfung einer Navigationsgenauigkeit des medizinischen AR-Systems 1 gemäß einer bevorzugten Ausführungsform der vorliegenden Offenbarung.

In einem ersten Schritt S1 wird zumindest ein aktueller Referenzpunkt 18 auf einen Patienten 10 präoperativ gesetzt. Beispielsweise erfolgt dies mithilfe eines medizinischen Markers. In einem zweiten Schritt S2 wird eine präoperative 3D-Aufnahme des Patienten 10 mit dem zumindest einen aktuellen Referenzpunkt 18 durch eine Visualisierungseinheit 2 erzeugt und bereitgestellt. Anschließend wird im dritten Schritt zumindest ein digitalisierter Referenzpunkt 20 aus dem zumindest einen aktuellen Referenzpunkt 18 der präoperativen 3D-Aufnahme des Patienten 10 durch die Steuereinheit 16 generiert und abgespeichert. Gleichzeitig, vor oder alternativ nach dem Schritt S3 wird im vierten Schritt S4 eine zeitaktuelle Aufnahme des Patienten 10 mit dem zumindest einen aktuellen Referenzpunkt 18 durch die Visualisierungseinheit 2 erzeugt und bereitgestellt. Anschließend wird im fünften Schritt eine AR-Überlagerungsdarstellung mit dem zumindest einen digitalisierten Referenzpunkt 20 und der zeitaktuellen Aufnahme des Patienten 10, welche den zumindest einen aktuellen Referenzpunkt 18 aufweist, erzeugt und im sechsten Schritt S6 auf der visuellen Darstellungsvorrichtung 14 ausgegeben. Im letzten Schritt S7 vergleicht die Steuereinheit 16 den zumindest einen digitalisierten Referenzpunkt 20 mit dem zumindest einen aktuellen Referenzpunkt 18 der zeitaktuellen Aufnahme.

In einem weiteren Schritt S8 kann ein Navigationsfehler 24 des medizinischen AR-Systems 1 aus einem Abstand zwischen dem zumindest einen digitalisierten Referenzpunkt 20 und dem zumindest einen aktuellen Referenzpunkt 18 der zeitaktuellen Aufnahme durch die Steuereinheit 16 berechnet werden.

In einer weiteren bevorzugten Ausführungsform der Offenbarung kann das Navigationssystem 8 eine Position und Orientierung einer medizinischen Instrumentenspitze 21 in einem globalen Koordinatensystem gegenüber dem Patienten 10 erfassen und der Schritt S3 Generieren und Abspeichern des zumindest einen digitalisierten Referenzpunkts 20 kann mithilfe der Position und Orientierung der medizinischen Instrumentenspitze 21 durchgeführt werden.

### Bezugszeichenliste

- 1: medizinisches AR-System
- 2: Visualisierungseinheit
- 4: beweglicher Roboterarm
- 6: medizinischer Roboter
- 8: Navigationssystem
- 10: Patient
- 12: Nutzer
- 14: Darstellungsvorrichtung
- 16: Steuereinheit
- 17: medizinisches Instrument
- 18: aktueller Referenzpunkt
- 20: digitalisierte Referenzpunkt
- 21: Instrumentenspitze
- 22: medizinische Plane
- 24: Navigationsfehler

S1 Schritt Setzen des zumindest einen aktuellen Referenzpunkts
S2 Schritt Erzeugen und Bereitstellen einer präoperativen 3D-Aufnahme
S3 Schritt Generieren und Abspeichern zumindest eines digitalisierten Referenzpunkts
S4 Schritt Erzeugen und Bereitstellen einer zeitaktuellen Aufnahme des Patienten
S5 Schritt Erzeugen einer AR-Überlagerungsdarstellung
S6 Schritt Ausgeben der AR-Überlagerungsdarstellung durch die Darstellungsvorrichtung
S7 Schritt Vergleichen des zumindest einen digitalisierten Referenzpunkts mit dem zumindest einen aktuellen Referenzpunkt
S8 Schritt Berechnen eines Navigationsfehlers des medizinischen AR-Systems

## Patentansprüche

1. Medizinisches AR-System (1) für einen chirurgischen, insbesondere neurochirurgischen, Eingriff eines Patienten (10), aufweisend:
eine Visualisierungseinheit (2), die eine präoperative Aufnahme des Patienten (10) mit zumindest einem auf diesem markierten Referenzpunkt (18) und eine zeitaktuelle Aufnahme des Patienten (10) mit dem zumindest einen Referenzpunkt (18) erzeugt und bereitstellt,
ein Navigationssystem (8), welches dafür ausgebildet ist, eine Position und Orientierung der Visualisierungseinheit (2) in einem globalen Koordinatensystem gegenüber dem Patienten (10) zu erfassen,
eine visuelle Darstellungsvorrichtung (14), um visuelle Informationen für den chirurgischen Eingriff darzustellen,
eine Steuereinheit (16), welche eingerichtet und vorbereitet ist, zumindest einen auf den Patienten (10) registrierten digitalisierten Referenzpunkt (20) aus dem zumindest einen Referenzpunkt (18) der präoperativen Aufnahme des Patienten (10) zu generieren und abzuspeichern,
**dadurch gekennzeichnet, dass**
die Steuereinheit (16) zusätzlich eingerichtet und vorbereitet ist, eine AR-Überlagerungsdarstellung mit dem zumindest einen digitalisierten Referenzpunkt (20) und der zeitaktuellen Aufnahme des Patienten (10) zu erzeugen, die AR-Überlagerungsdarstellung auf der visuellen Darstellungsvorrichtung (14) darzustellen, und den zumindest einen digitalisierten Referenzpunkt (20) mit dem zumindest einen Referenzpunkt (18) der zeitaktuellen Aufnahme zu vergleichen.

2. Medizinisches AR-System (1) nach Anspruch 1, **dadurch gekennzeichnet, dass** die Steuereinheit (16) eingerichtet und vorbereitet ist, einen Abstand zwischen dem zumindest einen digitalisierten Referenzpunkt (20) und dem zumindest einen Referenzpunkt (18) der zeitaktuellen Aufnahme als einen Navigationsfehler (24) des medizinischen AR-Systems (1) zu ermitteln und vorzugsweise automatisch zu korrigieren.

3. Medizinisches AR-System (1) nach Anspruch 1 oder 2, **dadurch gekennzeichnet, dass** das Navigationssystem (8) ausgebildet ist, eine Position und Orientierung einer medizinischen Instrumentenspitze (21) in einem globalen Koordinatensystem gegenüber dem Patienten (10) zu erfassen und die Steuereinheit (16) eingerichtet und ausgebildet ist, den zumindest einen digitalisierten Referenzpunkt (20) mithilfe der Position und Orientierung der medizinischen Instrumentenspitze (21) zu generieren.

4. Medizinisches AR-System (1) nach einem der Ansprüche 1 bis 3 **dadurch gekennzeichnet, dass** die Steuereinheit (16) eingerichtet und ausgebildet ist, den zumindest einen digitalisierten Referenzpunkt (20) mithilfe eines Laserpointers und/oder eines Fokuspunkts der Visualisierungseinheit (2) zu generieren.

5. Medizinisches AR-System (1) nach einem der Ansprüche 1 bis 4, **dadurch gekennzeichnet, dass** die Steuereinheit (16) eingerichtet und ausgebildet ist, den zumindest einen digitalisierten Referenzpunkt (20) mithilfe des zumindest einen Referenzpunkts (18) der von der Visualisierungseinheit (2) erzeugten präoperativen Aufnahme des Patienten (10) automatisch zu generieren.

6. Medizinisches AR-System (1) nach einem der vorstehenden Ansprüche, **dadurch gekennzeichnet, dass** das medizinische AR-System (1) ein AR-Headset und/oder ein 3D-Monitor und/oder ein VR-Headset als die Darstellungsvorrichtung (14) aufweist.

7. Medizinisches AR-System (1) nach einem der vorstehenden Ansprüche, **dadurch gekennzeichnet, dass** das Navigationssystem (8) ein infrarotbasiertes Trackingsystem oder ein elektromagnetisches Trackingsystem oder ein optisches Machine-Vision Trackingsystem aufweist.

8. Verfahren zur Überprüfung einer Navigationsgenauigkeit eines medizinischen AR-Systems (1), insbesondere nach einem der Ansprüche 1 bis 7, **gekennzeichnet durch** die Schritte:
- Setzen (S1) zumindest eines Referenzpunkts (18) auf einen Patienten (10);
- Erzeugen und Bereitstellen (S2) einer präoperativen Aufnahme des Patienten (10) mit dem zumindest einen Referenzpunkt (18) durch eine Visualisierungseinheit (2);
- Generieren und Abspeichern (S3) zumindest eines digitalisierten Referenzpunkts (20) aus dem zumindest einen Referenzpunkt (18) der präoperativen Aufnahme des Patienten (10), durch eine Steuereinheit (16);
- Erzeugen und Bereitstellen (S4) einer zeitaktuellen Aufnahme des Patienten (10) mit dem zumindest einen Referenzpunkt (18) durch die Visualisierungseinheit (2);
- Erzeugen (S5) einer AR-Überlagerungsdarstellung mit dem zumindest einen digitalisierten Referenzpunkt (20) und der zeitaktuellen Aufnahme des Patienten (10);
- Ausgeben (S6) der AR-Überlagerungsdarstellung durch eine visuelle Darstellungsvorrichtung (14); und
- Vergleichen (S7) des zumindest einen digitalisierten Referenzpunkts (20) mit dem zumindest einen Referenzpunkt (18) der zeitaktuellen Aufnahme durch die Steuereinheit (16).

9. Verfahren nach Anspruch 8, **gekennzeichnet durch** einen zusätzlichen Schritt
Berechnen (S8) eines Navigationsfehlers (24) des medizinischen AR-Systems (1) aus einem Abstand zwischen dem zumindest einen digitalisierten Referenzpunkt (20) und dem zumindest einen Referenzpunkt (18) der zeitaktuellen Aufnahme durch die Steuereinheit (16).

10. Verfahren nach Anspruch 8 oder 9, **dadurch gekennzeichnet, dass** ein Navigationssystem (8) eine Position und Orientierung einer medizinischen Instrumentenspitze (21) in einem globalen Koordinatensystem gegenüber dem Patienten (10) erfasst und der Schritt Generieren und Abspeichern (S3) des zumindest einen digitalisierten Referenzpunkts (20) mithilfe der Position und Orientierung der medizinischen Instrumentenspitze (21) durchgeführt wird.

11. Computerlesbares Speichermedium, umfassend Befehle, die bei einer Ausführung durch einen Computer diesen veranlassen, die Verfahrensschritte gemäß einem der Ansprüche 8 bis 10 auszuführen.
